# EUROPEAN PATENT APPLICATION

(11) **EP 3 907 238 A1**
(43) Date of publication of application: **10.11.2021**
(21) Application number: 20173303.7
(22) Date of filing: 06.05.2020
(51) Int. Cl.: C07K 16/00, C07K 16/12

(54) **METHOD FOR ISOLATING AN IMMUNOGLOBULIN A FRACTION AND USE OF SUCH FRACTION**

(71) Applicant: PreviPharma Consulting GmbH, 68167 Mannheim (DE)
(72) Inventor: Kießig, Stephan T., 69168 Wiesloch (DE); Mandago, Maurice, 69250 Schönau (DE); Welz, Ricarda, 69256 Mauer (DE); Gerding, Hanne Rieke, 68229 Mannheim (DE)
(74) Representative: Jacobi, Markus Alexander

(57) **Abstract**

The present invention relates to a method for isolating a fraction of immunoglobulin A (IgA) specifically binding to one or more carbohydrates specific for the surface of one or more bacteria species from a body fluid. Furthermore, the present invention refers to a fraction of IgA obtainable from such method and its use for treating or preventing a respiratory disease associated with an infection with *Haemophilus influenzae, Streptococcus pneumoniae, Neisseria meningitidis,* or a combination of two or more thereof.

## Description

The invention relates to a method for isolating a fraction of immunoglobulin A (IgA) specifically binding to one or more carbohydrates specific for the surface of one or more bacteria species from a body fluid. Furthermore, the present invention refers to a fraction of IgA obtainable from such method and its use for treating or preventing a respiratory disease associated with an infection with *Haemophilus influenzae* (*H. influenzae*), *Streptococcus pneumoniae* (*S. pneumoniae*), *Neisseria meningitides* (*N. meningitides*), or a combination of two or more thereof.

Infections with bacteria, in particular in immunocompromised or even immunodeficient patients, are still of concern in today's medicine. Such infections with bacteria, especially in immunocompromised or even immunodeficient patients, often result in chronic infections which may lead to death within a few years (Busse et al., Efficacy of intravenous immunoglobulin in the prevention of pneumonia in patients with common variable immunodeficiency. J Allergy Clin Immunol 2002; 109:1001-1004; Quinti et al., Polyvalent Immunoglobulins: Challenges and Perspectives. Blood Transfus, 11 Suppl 4 (Suppl 4), s40, 4 Sep 2013; Pulvirenti et al., Risk Factors for Haemophilus Influenzae and Pneumococcal Respiratory Tract Colonization in CVID. J Allergy Clin Immunol, 142(6):1999-2002.e3 Dec 2018).

Treatment with conventional antibiotics is often not sufficient, as the germs are often located on the mucosal surface of the upper respiratory tract where they cannot be sufficiently reached easily by many antibiotic drugs. One of the major defense mechanisms in healthy individuals is based on the IgA which is transported from B cells in the lymph nodes or the mucosa itself via epithelial cells onto the surface of bronchi, the trachea, nasal and paranasal sinuses. During this transport, IgA is - in particular in the gastrointestinal tract - often bound to the secretory component, which makes the IgA more protease resistant. One of the major functions of the IgA is the inhibition of binding of pathogens like viruses and bacteria to cells in the mucosa. Therefore, IgA may protect those cells / areas for getting infected and the infection is stopped.

This mechanism is diminished in immunocompromised and immunodeficient patients such as patients with primary immunodeficiencies (PIDs) such as, e.g., X-linked agammaglobulinemia, severe combined immunodeficiency (SCID), etc., and/or secondary immunodeficiencies (e.g. after treatment of leukemia patients with monoclonal anti-B-cell antibodies (e.g. anti-CD19, anti-CD20 - rituximab, anti-CD22, etc.) which may exhibit only reduces levels of IgA or lack IgA completely.

In patients suffering from an immune deficiency such as, e.g., CVID (common variable immune deficiencies) respiratory infections which are often predominantly due to bacteria such as *Streptococcus pneumoniae, Haemophilus influenzae* and, *Neisseria meningitides* are often of serious concern. Despite IgG replacement, recurrent respiratory infections are common in CVID, possibly leading to chronic lung damage (Quinti et al., Effectiveness of immunoglobulin replacement therapy on clinical outcome in patients with primary antibody deficiencies: results from a multicenter prospective cohort study, J Clin Immunol, 2011, 31:315-322) and poor quality of life (Quinti et al., Development and initial validation of a questionnaire to measure health-related quality of life of adults with common variable immune deficiency: the CVID_QoL Questionnaire, J Allergy Clin Immunol Pract, 2016, 4:1169-1179.e4).

Thus, patients are often prescribed antibiotics and/or long-term antimicrobial prophylactic regimens. Several regimens are used including rotation or periodically changing antibiotics (Kuruvilla and de la Morena, Antibiotic prophylaxis in primary immune deficiency disorders, J Allergy Clin Immunol Pract, 2013, 1:573-582). However, antibiotics may influence antimicrobial resistance among airway microbiota. In a recent meta-analysis on patients with chronic lung diseases, 30% of *S. pneumoniae* showed resistance to macrolides (Cameron et al., Long-term macrolide treatment of chronic inflammatory airway disease: risks, benefits and future developments, Clin Exp Allergy, 2012, 42:1302-1312; Pulvirenti et al., Risk Factors for Haemophilus Influenzae and Pneumococcal Respiratory Tract Colonization in CVID, 2018, J Allergy Clin Immunol, 142(6):1999-2002.e3). Risk factors related to colonization were initially identified by Pulvirenti et al. using a binary logistic regression.

Quinti et al. showed 2011 that overall, 21% of the patients with common variable immunodeficiencies and 24% of patients with X-linked agammaglobulinemia remained infection free during the study.

A reduction of pneumonia episodes has been observed after initiation of immunoglobulin G (IgG) replacement. A replacement of IgA was not possible due to the unavailability of such a product. In X-linked agammaglobulinemia, a co-morbidity risk factor identified for pneumonia by the final multivariable model was the presence of bronchiectasis.

Low IgM and low IgA serum levels were found to be risk factors for *S. pneumoniae* and *H. influenzae* colonization. Colonization by culture was associated with IgM serum levels of less than 5 mg/dL (odds ratio [OR], 3.7; 95% CI (confidence interval), 0.86-15.99; P 5.05; OR, 2.5; 95% CI, 0.95-6.45; P 5.05, respectively) and with IgA serum levels of less than 7 mg/dL (OR, 5.7; 95% CI, 0.69-47.17; P 5 .05; OR, 8.9; 95% CI, 1.92-40.84; P 5.001, respectively). Patients co-colonized often have particularly low IgA and IgM serum levels (1.3 to 0.7 mg/dL and 1.7 to 1.2 mg/dL, respectively). These data were confirmed in patients with hyper-IgM syndrome and healthy subjects where IgM antibodies have a distinct anti-*H. influenzae* activity in the prevention of respiratory tract bacterial colonization (Micol et al., Protective effect of IgM against colonization of the respiratory tract by nontypeable Haemophilus influenzae in patients with hypogammaglobulinemia, J Allergy Clin Immunol, 2012, 129:770-777; Choi et al., Naturally occurring bactericidal antibodies specific for Haemophilus influenzae lipooligosaccharide are present in healthy adult individuals, 2015, Vaccine 33:1941-1947; Barra et al., Characterization of the serum antibody response induced by Haemophilus influenzae type b tetanus protein-conjugate vaccine in infants receiving a DTP-combined vaccine from 2 months of age, 1993, Vaccine 11:1003-1006).

The actual difficulties to replace IgA and IgM at the mucosal level implied the need to consider additional therapeutic interventions in primary antibody deficiencies, such as the use of antibiotic treatment or prophylaxis. However, a major concern related to the use of antibiotic therapy or prophylaxis is that it exerts selective pressure on airway microbiota, facilitating the emergence of antibiotic resistance as shown by Pulvirenti et al. (Risk Factors for Haemophilus Influenzae and Pneumococcal Respiratory Tract Colonization in CVID, J Allergy Clin Immunol, 142(6):1999-2002.e3 Dec 2018)

In some immunodeficiencies, it was possible to identify a clinical phenotype characterized by a high pneumonia risk: patients with low IgG and IgA levels at diagnosis; patients who had IgA level <7 mg/dL and who had bronchiectasis. (Quinti et al.: Effectiveness of Immunoglobulin Replacement Therapy on Clinical Outcome in Patients with Primary Antibody Deficiencies: Results from a Multicenter Prospective Cohort Study. J Clin Immunol, 2011, 31:315-322). Despite of the immunoglobulin G substitution, there is still a high risk for infections of the upper respiratory tract in those patients with a high risk of death within the next three years.
Immunoglobulins like IgG and IgA have already been proven in their efficacy against a number of respiratory infections, either caused by bacteria or viruses like RSV (respiratory syncytial virus) (Weltzin et al., Intranasal Monoclonal Immunoglobulin A against Respiratory Syncytial Virus Protects against Upper and Lower Respiratory Tract Infections in Mice, 1994, Antimicobial Agents and Chmotherapy 38(12):2785-2791).

IgA was also found to have some effect on *Streptococcus pneumoniae* (Janoff et al., The Journal of Clinical Investigation, 1999, 104:1139-1147; Janoff et al., Mucosal Immunol., 2014, 7(2):249-256; Vonarburg et al. Respiratory Research, 2029, 20:99). However, sufficiently purely isolated and concentrated fractions of IgA are hardly obtainable. Impurities with IgG or with IgA having other binding profiles may harm the effectivity.

There is still an unmet need for compounds that are able to specifically irradiate bacteria, in particular in the respiratory tract.

Surprisingly, it was found that bacteria-specific immunoglobulin A (IgA) can be isolated from body fluids by means of using a solid support containing one or more immobilized carbohydrates to which the IgA specifically binds to and a further step of separating IgA from other immunoglobulins, in particular from immunoglobulin G (IgG).

Accordingly, an aspect, the present invention relates to a method for isolating a fraction of immunoglobulin A (IgA) specifically binding to one or more carbohydrates specific for the surface of one or more bacteria species from a body fluid, wherein the method comprises the following steps:
(i) providing:
   the body fluid containing one or more immunoglobulins specifically binding to the one or more carbohydrates; and
   a solid support containing one or more immobilized carbohydrates;
(ii) contacting the body fluid of step with the solid support and allowing binding of the one or more of immunoglobulins to the one or more carbohydrates immobilized on the solid support,
(iii) removing the remaining body fluid containing components not bound to the one or more carbohydrates immobilized on the solid support in step (ii) from the solid support and optionally washing the solid support; and
(iv) eluting the one or more of immunoglobulins specifically binding to the one or more carbohydrates immobilized on the solid support from the solid support,
wherein the method further comprises at least one step of separating IgA from other immunoglobulins, in particular from immunoglobulin G (IgG).

As used herein, the term "carbohydrate" may be understood in the broadest sense as generally understood in the art. A carbohydrate may also be designated as a saccharide (also Polysaccharide or oligosaccharide).

The term fraction of IgA may be understood in the broadest sense as a selected stake of the IgA pool of a body fluid. It should not be misunderstood as a shortened or degraded IgA. In a preferred embodiment, IgA is full-length IgA including light and heavy chains.

In a preferred embodiment, the method further comprises the step of immobilizing the one or more carbohydrates on a solid support.

As used herein, the terms "solid support", "solid phase", and "matrix" may be understood in the broadest sense as generally understood in the art. For example, the solid support may be particular or may be a monolith. Preferably, the solid support is particulate. Preferably, the solid support is spherical in a mean size range of 0.001 to 2 mm or 0.01 to 1 mm.

In a preferred embodiment, the solid support containing one or more immobilized carbohydrates is located in an affinity column. Thus, in a preferred embodiment, steps (ii) to (iii) are conducted in an affinity column. Contacting the body fluid of step with the solid support and allowing binding of the one or more of immunoglobulins to the one or more carbohydrates immobilized on the solid support /step (ii)) may be achieved by a flow through a column or by suspending a particulate solid phase in the body fluid.

Removing the remaining body fluid from the solid support (step (iii)) may be conducted by any means. For example, in an affinity column, the buffer may be changed to a buffer not containing or containing low contents of the body fluid and thereby washing away the remaining unbound components of the body fluid. Alternatively, suspended solid phase may also be removed by centrifugation, filtration, precipitation, or combinations of two or more thereof. Washing may be performed by a suitable buffer, typically maintaining integrity of the bound IgA. Such buffer preferably does not impede binding of the IgA to the carbohydrates.

Eluting the one or more of immunoglobulins (step (iv)) may be performed by any means. For example, salt concentrations may be altered accordingly as known by the person skilled in the art.

The body fluid may be any body fluid. In a preferred embodiment, the body fluid is blood plasma or a fraction thereof. Blood plasma and fractions thereof may be obtained from any source. Such plasma may be obtained from naturally immune donors or from immunized or even (hyper)immunized donors. Alternatively or additionally, the body fluid may also contain one or more recombinant components. Alternatively or additionally, the body fluid may also contain one or more monoclonal or polyclonal immunoglobulins, including IgA.

As immunoglobulin (e.g. IgA) may also be considered as an antibody (e.g., an IgA antibody).

In a preferred embodiment, the fluid is a body fluid, preferably a body fluid selected from the group consisting of blood plasma and a fraction of blood plasma, in particular wherein said method further comprises preparing of a fraction of blood plasma by means of a Cohn or Kistler-Nitschmann process.

Purification of immunoglobulins by the Cohn process (Cohn et al., Preparation and Properties of Serum and Plasma Proteins. IV. A System for the Separation into Fractions of the Protein and Lipoprotein Components of Biological Tissues and Fluids, 1946, Journal of the American Chemical Society 68 (3): 459-475) and/or Kistler-Nitschmann process (Kistler and Nitschmann, Large Scale Production of Human Plasma Fractions. Eight Years Experience with the Alcohol Fractionation Procedure of Nitschmann, Kistler and Lergier, 1962, Vox Sang 7:414) is known in the art.

In a preferred embodiment, the body fluid is the IgA/IgM fraction of Cohn fractionation and optionally further removing immunoglobulin M (IgM).

Blood plasma may be obtained from a single donor or may be a pooled fraction of blood plasma samples.

The patient may be untreated or may be immunized. In a preferred embodiment, the body fluid containing one or more of immunoglobulins specifically binding to the one or more carbohydrates is obtained from a subject previously exposed to the one or more carbohydrates, optionally to the one or more bacteria species bearing the one or more carbohydrates on their surface.

For example, the subject is exposed to the one or more carbohydrates (and optionally the bacteria carrying such) at least one week, between one week and a year, between one and two weeks, between one week and a month, between two weeks and a month, between two weeks and two months or between one and six months. As used in the context of the present invention, the term "subject" may be understood in the broadest sense as any living being, which is preferably any animal, more preferably a mammal including human, in particular a human being. A subject from which plasma is obtained from may also be considered and designated as (plasma) donor.

The body fluid, in particular blood plasma or a fraction thereof, may be obtained from a subject (plasma donor) subjected to (hyper)lmmunization. Alternatively or additionally, selection of the subject (plasma donor) may also involve using commercial vaccines and, optionally, a test system allowing the differentiation between high- and low tittered plasma/blood donations.

An S. *pneumoniae* vaccine may be any such vaccine known in the art. There are several types of pneumococcal vaccines: conjugate vaccines and polysaccharide vaccines. They are given by injection either into a muscle or just under the skin (World Health Organization, 2012, "Pneumococcal vaccines: WHO position paper - 2012". Wkly Epidemiol Rec. 87(14):129-144. hdl:10665/241904. PMID 24340399. Lay summary). A polysaccharide vaccine may be Pneumovax 23 (MSD) containing purified polysaccharides from 23 serotypes (1, 2, 3, 4, 5, 6b, 7F, 8, 9N, 9V, 10A, 11A, 12F, 14, 15B, 17F, 18C, 19A, 19F, 20, 22F, 23F, and 33F) (Pletz et al., Pneumococcal vaccines: mechanism of action, impact on epidemiology and adaption of the species, 2008, Int. J. Antimicrob. Agents. 32(3):199-206. doi:10.1016/j.ijantimicag.2008.01.021. PMID 18378430; Stein, Thymus-independent and thymus-dependent responses to polysaccharide antigens, 1992, J. Infect. Dis. 165(Suppl 1):S49-S52).

A *H. influenzae* type B vaccine, often called HIB vaccine is also usable in the context of the present invention. The antigens are often present in combination vaccines like Hexyon® Injektionssuspension, Infanrix hexa, Pentavac®, Vaxelis®, etc. A vaccine against *N. meningitis* is often included in combination vaccines available on the market. Examples are Menveo® or Nimenrix®. All three antigens in the commercially available vaccines are effective in healthy individuals for the induction of protective antibodies. The plasma of those immunized individuals, especially the immunoglobulin fractions can be used for a passive immunization (transfer of specific antibody by e.g. the parenteral application of specific antibodies) for individuals having no protective immune responses. Surprisingly, the passive transfer of specific IgA on the mucosal surface is also protective by reducing the colonization with *H. influencae, S. pneumoniae* and *N. meningitidis* of the mucosal surfaces in the upper respiratory tract.

Other immunoglobulins than IgA may be any other types of immunoglobulins (e.g., IgG, IgM, IgD, IgE). In a preferred embodiment, one or more other immunoglobulins than IgA may be selected from the group consisting of immunoglobulin G (IgG), immunoglobulin M (IgM), immunoglobulin D (IgD), immunoglobulin E (IgE), and combinations of two or more thereof. In a preferred embodiment, IgA is separated from IgG and optional further immunoglobulins. In a preferred embodiment, IgA is separated from IgM and optional further immunoglobulins. In a preferred embodiment, IgA is separated from IgG and IgM and optional further immunoglobulins

The at least one step of separating IgA from other immunoglobulins may be conducted by any means. In a preferred embodiment, the step of separating IgA from other immunoglobulins may be conducted by mans of affinity chromatography. In a preferred embodiment, the step of separating IgA from other immunoglobulins may be conducted by mans of affinity chromatography wherein one or more agents (affinity ligands) specifically binding to IgA (e.g., one or more IgA-specific antibodies or antibody-fragments) are immobilized on a solid support.

Then, the fluid of interest containing IgA and optionally other immunoglobulins may be contacted with the solid support allowing binding of the IgA specifically to the immobilized one or more agents (affinity ligands) specifically binding to IgA. The remaining fluid may be removed. The solid support to which IgA is bound may optionally be washed (e.g., with a buffer). In a further step, the IgA may be eluated from the solid support.

An agent specifically binding to IgA (affinity ligand) may be any agent. For example, it may be *CaptureSelect*™ *IgA* or *CaptureSelect*™ *IgA-CH1* (Thermo-Fisher, Germany) or any other IgA-specific ligand being either a whole protein or just a peptide fragment, like a Fab fragment.

Additionally or alternatively, the step of at least one step of separating IgA from other immunoglobulins may involve further procedures such as precipitation (e.g., with ammonium sulfate), size exclusion chromatography, blood plasma fractionation (e.g., Cohn fractionation), ion-exchange (IEX) chromatography, nanofiltration, or a combination of two or more thereof. In a preferred embodiment, at least two or three thereof are combined with each other.

In a preferred embodiment, the step of at least one step of separating IgA from other immunoglobulins may involve precipitation (e.g., with ammonium sulfate), size exclusion chromatography, and blood plasma fractionation (e.g., Cohn fractionation).

The at least one step of separating IgA from other immunoglobulins may be conducted at any time of the method of the present invention. It may be conducted before, after or while the body fluid is contacted with the solid support.

In a preferred embodiment, the at least one step of separating IgA from other immunoglobulins comprises or consists of separating IgA from IgG. In a preferred embodiment, the at least one step of separating IgA from other immunoglobulins comprises or consists of separating IgA from IgM. In a preferred embodiment, the at least one step of separating IgA from other immunoglobulins comprises or consists of separating IgA from IgD. In a preferred embodiment, the at least one step of separating IgA from other immunoglobulins comprises or consists of separating IgA from IgE. In a preferred embodiment, the at least one step of separating IgA from other immunoglobulins comprises or consists of separating IgA from IgG and IgM.

In a preferred embodiment, the at least one step of separating IgA from other immunoglobulins comprises or consists of separating IgA from IgG, IgM, IgD and IgE.

Separating IgA from IgG and IgM and optionally further immunoglobulins may be conducted in a single step or two or more separate steps.

In a preferred embodiment, the at least one step of separating IgA from other immunoglobulins is conducted before the body fluid is contacted with the solid support. In this context, in a preferred embodiment, the body fluid is the IgA/IgM fraction of Cohn fractionation and optionally further removing immunoglobulin M (IgM).

The IgA/IgM fraction of Cohn fractionation may also be considered as the IgM and IgA waste fraction which may generated during the polishing of IgG products in Cohn Fractionation. The IgA in this fraction may be optionally separated using ion-exchange (IEX) chromatography and/or nanofiltration to remove IgM and purify IgA.

Removing IgM (i.e., separating IgA and IgA) may be performed by any means known in the art. In a preferred embodiment, removing IgM (i.e., separating IgA and IgA) may be performed by affinity chromatography specific for IgA.

In a preferred embodiment, removing IgM (i.e., separating IgA and IgA) may be performed by affinity chromatography specific for IgA, wherein one or more agents specifically binding to IgA (e.g., one or more IgA-specific antibodies or antibody-fragments) are immobilized on a solid support. Then, the fluid of interest containing IgA and IgM may be contacted with the solid support allowing binding of the IgA specifically to the immobilized one or more agents (affinity ligands) specifically binding to IgA ((essentially not binding to IgM). The remaining fluid may be removed. The solid support to which IgA is bound may optionally be washed (e.g., with a buffer). In a further step, the IgA may be eluated from the solid support.

The purification of specific IgA is enabled as the polysaccharide-based epitopes recognized by the specific immunoglobulins against *H. influenzae* and/or S. *pneumonia* and/or *N. meningitides* are used insolubilized for affinity chromatography.

In an alternative preferred embodiment, removing IgM (i.e., separating IgA and IgA) may be performed by affinity chromatography specific for IgM, wherein one or more agents (affinity ligands) specifically binding to IgM (e.g., one or more IgM-specific antibodies or antibody-fragments) are immobilized on a solid support. Then, the fluid of interest containing IgA and IgM may be contacted with the solid support allowing binding of the IgM specifically to the immobilized one or more agents (affinity ligands) specifically binding to IgM ((essentially not binding to IgA). The desired IgA is maintained in the eluate (flow-through).

Additionally or alternatively, the step of removing IgM (i.e., separating IgA and IgA) may involve further procedures such as precipitation (e.g., with ammonium sulfate), size exclusion chromatography, blood plasma fractionation (e.g., Cohn fractionation), ion-exchange (IEX) chromatography, nanofiltration, or a combination of two or more thereof.

In a preferred embodiment, the at least one step of separating IgA from other immunoglobulins is conducted in the eluate obtained in step (v).

This step may be conducted by any means such as described above. In a preferred embodiment, the eluate subjected to known fractionation processes and optionally further removing immunoglobulin M (IgM) (Cohn et al., Preparation and Properties of Serum and Plasma Proteins. IV. A System for the Separation into Fractions of the Protein and Lipoprotein Components of Biological Tissues and Fluids, 1946, Journal of the American Chemical Society 68(3):459-475; Kistler and Nitschmann, Large Scale Production of Human Plasma Fractions. Eight Years Experience with the Alcohol Fractionation Procedure of Nitschmann, Kistler and Lergier, 1962, Vox Sang 7:414).

The carbohydrates may be any surface carbohydrates of bacteria, in particular bacteria associated with respiratory diseases in particular diseases of the upper respiratory tract. Such respiratory disease may be caused by streptococci , pneumococci or both. In a preferred embodiment, a respiratory disease may be selected from the group consisting of pharyngitis, laryngitis, tracheitis, bronchitis, sinusitis, and a combination of two or more thereof.

As used herein, the term "surface carbohydrates of bacteria" my be understood in the broadest sense as carbohydrates that are of such type found on the surface of bacteria. These carbohydrates may be obtained from bacteria. However, the carbohydrates are not necessarily obtained from bacteria. These can also be obtained form any other source such as from chemical synthesis, from recombinant organisms, biotechnological means, or combinations ow two or more thereof. In the later case, the carbohydrates are structurally of the type found on the surface of bacteria.

In a preferred embodiment, the one or more carbohydrates are surface carbohydrates of *Haemophilus influenzae, Streptococcus pneumoniae, Neisseria meningitides,* or a combination of two or more thereof.

In a preferred embodiment, the one or more carbohydrates are polyribosyl ribitol phosphate (PRP). In a preferred embodiment, the one or more carbohydrates are polyribosyl ribitol phosphate (PRP) containing 5 to 50 units of PRP moieties.

In another preferred embodiment, the one or more carbohydrates are those found on *Haemophilus influenzae surfaces* as described in Baek et al. (Chem. Sci, 2018, 9:1279-1288).

In another preferred embodiment, the one or more carbohydrates are those found on *Streptococcus pneumoniae surfaces* as described in Ménová et al. (supportive Material of Identification of the Minimal Glycotope of Streptococcus pneumoniae 7F Capsular Polysaccharide using Synthetic Oligosaccharides, Chemistry - A European Journal, 2018, 24(16):4181-4187), in Lin et al. (Structure elucidation of capsular polysaccharides from Streptococcus pneumoniae serotype 33C, 33D, and revised structure of serotype 33B, Carbohydr Res., 2014, 383:97-104), or in Geno et al., Clinical Microbiology Reviews , 2015, 28(3):871-899).

The carbohydrate may also be a commercially obtainable vaccine such as *H*. *influenzae* type B vaccine (HIB), Pneumovax (e.g., Pneumovax 23), Synflorix or Prevenar 13.

The bacteria on which surface the one or more carbohydrates originate from may be any bacteria.

In a preferred embodiment, the one or more bacteria species are bacteria associated with one or more respiratory diseases.

In a preferred embodiment, the one or more bacteria species are bacteria are selected from the group consisting of *Haemophilus influenzae, Streptococcus pneumoniae, Neisseria meningitides,* and a combination of two or more thereof.

In a preferred embodiment, a respiratory disease is a disease of the upper respiratory tract. In a preferred embodiment, a disease of the upper respiratory tract may be selected from the group consisting of pharyngitis, laryngitis, tracheitis, bronchitis, sinusitis, and a combination of two or more thereof.

The fraction of IgA bears unexpectedly beneficial technical characteristics in that it is particularly pure and target-specific.

A further aspect of the present invention thus relates to a fraction of IgA obtainable (obtained) from a method of the present invention.

It will be understood that all definitions are preferred embodiments as indicated in the context of the method above *mutatis mutandis* also apply to the fraction of IgA obtainable from such method.

In a preferred embodiment, the fraction of IgA is apyrogenic. The fraction of IgA may be provided in an ampoule.

The present invention also relates to a dosage unit of the fraction of IgA of the present invention. Exemplarily, the present invention may refer to a single dose container or to a multiple dosage form.

The fraction of IgA may be used as such for any purpose. The fraction of IgA may also be embedded in a pharmaceutical composition.

Accordingly, a further aspect of the present invention relates to a pharmaceutical composition comprising the fraction of IgA of the present invention and at least one pharmaceutically acceptable carrier.

It will be understood that all definitions are preferred embodiments as indicated above *mutatis mutandis* also apply to the pharmaceutical composition.

In a preferred embodiment, the pharmaceutical composition is a spray, an aerosol, a nose drop, an unguent, an ointment, a gel, or a microcapsule.

As used herein, the terms "pharmaceutically acceptable carrier", "pharmaceutically acceptable excipient", "carrier" and "excipient" may be understood interchangeably in the broadest sense as any substance that may support the pharmacological acceptance of the fraction of IgA.

A pharmaceutically acceptable carrier may exemplarily be selected from the list consisting of an aqueous buffer, saline, water, dimethyl sulfoxide (DMSO), ethanol, vegetable oil, paraffin oil or combinations of two or more thereof. Furthermore, the pharmaceutically acceptable carrier may optionally contain one or more detergent(s), one or more foaming agent(s) (e.g., sodium lauryl sulfate (SLS), sodium dodecyl sulfate (SDS)), one or more coloring agent(s) (e.g., food coloring), one or more vitamin(s), one or more salt(s) (e.g., sodium, potassium, calcium, zinc salts), one or more humectant(s) (e.g., sorbitol, glycerol, mannitol, propylenglycol, polydextrose), one or more enzyme(s), one or more preserving agent(s) (e.g., benzoic acid, methylparaben, one or more antioxidant(s), one or more herbal and plant extract(s), one or more stabilizing agent(s), one or more chelating agents (e.g., ethylenediaminetetraacetic acid (EDTA), and/or one or more uptake mediator(s) (e.g., polyethylene imine (PEI), a cell-penetrating peptide (CPP), a protein transduction domain (PTD), an antimicrobial peptide, etc.).

In a preferred embodiment, the pharmaceutical composition is apyrogenic. The pharmaceutical composition may be provided in an ampoule.

The present invention also relates to a dosage unit of the pharmaceutical composition of the present invention. Exemplarily, the present invention may refer to a single dose container or to a multiple dosage form.

As noted before, in a preferred embodiment, IgA specifically binds to one or more carbohydrates specific for the surface of one or more bacteria species selected from the group consisting of *Haemophilus influenzae, Streptococcus pneumoniae, Neisseria meningitides,* and a combination of two or more thereof. Such fraction of IgA or the pharmaceutical composition of the present invention may be used for any purpose, including prophylactic and therapeutic uses.

Accordingly, a further aspect of the present invention relates to the fraction of IgA or the pharmaceutical composition of the present invention, wherein the IgA specifically binds to one or more carbohydrates specific for the surface of one or more bacteria species selected from the group consisting of *Haemophilus influenzae, Streptococcus pneumoniae, Neisseria meningitides,* and a combination of two or more thereof,
for use in a method for treating or preventing a respiratory disease associated with an infection with *Haemophilus influenzae, Streptococcus pneumoniae, Neisseria meningitidis,* or a combination of two or more thereof.

It will be understood that all definitions are preferred embodiments as indicated in the context of the fraction of IgA and pharmaceutical composition above *mutatis mutandis* also apply to such compounds and compositions for use in a method for treating or preventing of the present invention

In other words, the present invention relates to a method for treating or preventing a respiratory disease associated with an infection with *Haemophilus influenzae, Streptococcus pneumoniae, Neisseria meningitidis,* or a combination of two or more thereof in a patient, wherein the patient is administered with a sufficient amount of the fraction of IgA or the pharmaceutical composition of the present invention.

Surprisingly, fractions of IgA of the present invention (optionally forming part of a pharmaceutical composition of the present invention) has the property to neutralize or inhibit the binding of *H. influenzae* and/or S. *pneumonia and*/*or N. meningitidis,* on the mucosal surface and prevent by this the infection of the patients via the upper respiratory tract. This may be realized by the binding of the specific IgA on the surface of the bacteria.

In a preferred embodiment, the present invention relates to the fraction of IgA or the pharmaceutical composition of the present invention, wherein the IgA specifically binds to one or more carbohydrates specific for the surface of *Haemophilus influenzae,* for use in a method for treating or preventing a respiratory disease associated with an infection with *Haemophilus influenzae.*

In a preferred embodiment, the present invention relates to the fraction of IgA or the pharmaceutical composition of the present invention, wherein the IgA specifically binds to one or more carbohydrates specific for the surface of *Streptococcus pneumoniae,* for use in a method for treating or preventing a respiratory disease associated with an infection with *Streptococcus pneumoniae.*

In a preferred embodiment, the present invention relates to the fraction of IgA or the pharmaceutical composition of the present invention, wherein the IgA specifically binds to one or more carbohydrates specific for the surface of *Neisseria meningitidis,* for use in a method for treating or preventing a respiratory disease associated with an infection with *Neisseria meningitidis.*

As used in the context of the present invention, the term "patient" may be understood in the broadest sense as any living being, which is preferably any animal, more preferably a mammal including human, in particular a human being. The patient may show symptoms of a respiratory disease or ay be healthy, but of risk of developing a respiratory disease (i.e., also reduce the infection risk).

The fraction of IgA or the pharmaceutical composition may be administered by any means. Any route of administration may be applied. For example, the fraction of IgA or the pharmaceutical composition may be administered by systemic administration (e.g., intravenously (i.v.), intraarterially (i.a.), intraperitoneally (i.p.), intramuscularly (i.m.), subcutaneously (s.c.), transdermal, nasally). Alternatively, administration may be local administration (e.g., intrathecally or intravitreally, intranasal, respiratory). Administration may be performed by inhalation or by dropping or spray.

In a preferred embodiment, the fraction of IgA or the pharmaceutical composition is administered to the patient's respiratory tract. In a preferred embodiment, the fraction of IgA or the pharmaceutical composition is administered as an aerosol. In a preferred embodiment, the fraction of IgA or the pharmaceutical composition is administered to the patient's respiratory tract as an aerosol. Administration as an aerosol may be understood in the broadest sense as any administration by means of breath. The terms "as aerosol" also include inhalation. Administering as aerosol typically includes inhalation after nebulization or by drops. The effect is stringer when the IgA is applied as aerosol than when the IgA was applied intravenously, intramuscular or subcutaneously.

The administration may be performed at any administration scheme. For example, administration may be performed by administering a volume of 0.01 to 5 mL, of 0.1 to 2 mL, or of 0.05 to 0.25 mL as aerosol to the patient.

The IgA concentration in a pharmaceutical composition may, for example, be in the range of 0.01 to 100 mg/mL, or 0.1 to 10 mg/mL.

For example, administration may be once every two weeks, once every week or twice daily (e.g. for prophylaxis). It may also be once, twice, 3 times, 4, times, 5 times, 6 times, 7 times, 8 times, 9 times or 10 times or even every hour or constantly (e.g., for treatment purposes).

For example, administration may be include administering 0.1 to 2 mL of a pharmaceutical composition of 0.1 to 100 mg/mL of IgA of the present invention, in particular by inhalation after nebulization or by drops (i.e., as aerosol).

For example, administration may be include administering 0.05 to 0.2 mL of a pharmaceutical composition of at least 1 mg/mL of IgA of the present invention, in particular by inhalation after nebulization or by drops (i.e., as aerosol) (e.g., once per week for prevention, 5 to 10 times per day for therapeutic purposes)

The patient may be a healthy patient or a patient suffering from an immune deficiency. In a preferred embodiment, the patient is immunocompromised or suffers from an immune deficiency. In a preferred embodiment, the patient suffers from an immune deficiency.

In a preferred embodiment, the patient suffers from an immune deficiency selected from the group consisting of X-linked agammaglobulinemia (XLA), common variable immunodeficiency (CVID), hyper-IgM syndrome with AID or UNG deficiencies, hyper-IgM syndrome with CD40 deficiency, hyper-IgM syndrome with CD40 ligand deficiency, selective antibody deficiency with normal immunoglobulins, selective IgA deficiency, transient hypogammaglobulinemia of infancy, ataxia-telangiectasia cartilage-hair hypoplasia, combined immunodeficiency with inadequate but not absent T-cell function and normal or elevated immunoglobulins, hyper-lgE syndrome, MHC antigen deficiencies, severe combined immunodeficiency, Wiskott-Aldrich syndrome, and combination of two or more thereof.

In a preferred embodiment, the patient suffers from one or more humoral immune deficiencies as listed in Table 1 below and/or one or more combined immune deficiencies as listed in Table 2 below.

**Table 1. Humoral immuno deficiencies**

| Disorder | Inheritance | Genes Affected | Clinical Findings |
|---|---|---|---|
| Common variable immunodeficiency | variable | *PIK3CD, BAFFR, CTLA4,* /*COS, TACI, NKFKB2, STAT3* | autoimmune disorders (e.g., immune thrombocytopenia, autoimmune hemolytic anemia), recurrent sinopulmonary infections, malabsorption, giardiasis, granulomatous interstitial lung disease, nodular lymphoid hyperplasia of the gastro intstinal tract, bronchiectasis, lymphocytic interstitial pneumonia, splenomegaly; in 10%, gastric carcinoma and lymphoma; usually diagnosed in patients aged 20-40 years |
| Selective antibody deficiency with normal immunoglobulins | unknown | - | recurrent sinopulmonary infections; sometimes atopic manifestations (e.g., atopic dermatitis, asthma, chronic rhinitis); may occur in mild, moderate, severe, and memory phenotypes |
| Selective IgA deficiency | Unknown | in some cases *TACl* | most often asymptomatic; recurrent sinopulmonary infections, diarrhea, allergies (including anaphylactic transfusion reactions (rare)), autoimmune disorders (e.g., celiac disease, inflammatory bowel disease, SLE, chronic active hepatitis) |
| Transient hypogamma-globulinemia of infancy | Unknown | - | usually asymptomatic; sometimes recurrent sinopulmonary or gastrointestinal infections, candidiasis, meningitis |
| X-linked agammaglobulinemia | X-linked | *BTK* | recurrent sinopulmonary and skin infections during infancy, transient neutropenia, lymphoid hypoplasia Persistent central nervous system (CNS) infections resulting from live-attenuated oral polio vaccine, echoviruses, or coxsackieviruses; increased risk of infectious arthritis, bronchiectasis, and certain cancers |
| Hyper-IgM syndrome with AID or UNG deficiencies | autosomal recessive | *AID, UNG* | similar to X-linked hyper-IgM syndrome, but with lymphoid hyperplasia; no leukopenia |
| Hyper-IgM syndrome with CD40 deficiency | autosomal recessive | *CD40* | similar to X-linked hyper-IgM syndrome; lymphoid hypoplasia, neutropenia |
| Hyper-IgM syndrome with CD40 ligand deficiency | X-linked | *CD40 igand (CD40L)* | similar to X-linked agammaglobulinemia (e.g., recurrent pyogenic bacterial sinopulmonary infections), but greater frequency of pneumocystis jirovecii pneumonia, cryptosporidiosis, severe neutropenia, and lymphoid hypoplasia |

| | | | |
|---|---|---|---|
| herein: AID = activation-dependent (induced) cytidine deaminase; BAFFR = B-cell activating factor receptor; BTK = Bruton tyrosine kinase; C = complement; CAML = calcium-modulator and cyclophilin ligand; CD = clusters of differentiation; CTLA4 = cytotoxic T-lymphocyte-associated protein 4; ICOS = inducible T-cell co-stimulator; NKFKB2 = nuclear factor kappa B subunit 2PIK3CD = phosphatidyl 3-kinase catalytic subunit delta; SLE = systemic lupus erythematosus; STAT3 = signal transducer and activator of transcription 3; TACI = transmembrane activator and CAML interactor; and UNG = uracil DNA glycosylase. | | | |

**Table 2. Combined immune deficiencies**

| Disorder | Inheritance | Genes Affected | Clinical Findings |
|---|---|---|---|
| Severe combined Immuno deficiency | Autosomal recessive or X-linked | *JAK3,* PTPRC (*CD45*), *RAG1, RAG2* (autosomal recessive) *IL-2RG* (X-linked) | oral candidiasis, *pneumocystis jirovecii* pneumonia, diarrhea before 6 months, failure to thrive, graft vs host disease, absent thymic shadow, lymphopenia, bone abnormalities (in ADA deficiency), exfoliative dermatitis as part of Omenn syndrome |
| Ataxia-telangiectasia | Autosomal recessive | *ATM* | ataxia, telangiectasias, recurrent sinopulmonary infections, endocrine abnormalities (e.g., gonadal dysgenesis, testicular atrophy, diabetes mellitus), increased risk of cancer |
| Cartilage-hair hypoplasia | Autosomal recessive | *RMRP* | short-limbed dwarfism, common and opportunistic infections |
| Combined Immuno deficiency with inadequate but not absent T-cell function and normal or elevated immune-globulins | Autosomal recessive or X-linked | *NEMO* | common and opportunistic infections, lymphopenia, lymphadenopathy, hepatosplenomegaly, skin lesions resembling those of Langerhans cell histiocytosis in some patients |
| Hyper-IgE syndrome | Autosomal dominant or recessive | *STAT3* (dominant) *TYK2, DOCK8* (recessive) | sinopulmonary infections; staphylococcal abscesses of skin, lungs, joints, and viscera; pulmonary pneumatoceles; pruritic dermatitis; coarse facial features; delayed shedding of baby teeth; osteopenia; recurrent fractures; tissue and blood eosinophilia |
| MHC antigen deficiencies | Autosomal recessive | various including, *RFX*: RFXANK, RFX5, and RFXAP | common and opportunistic infections |
| Wiskott-Aldrich syndrome | X-linked recessive | *WASP* | typically, pyogenic and opportunistic infections, eczema, thrombocytopenia; possibly gastrointestinal bleeding (e.g., bloody diarrhea), recurrent respiratory infections, cancer (in 10% of patients >10 years), varicella-zoster virus infection, herpesvirus infection |

| | | | |
|---|---|---|---|
| herein: ADA = adenosine deaminase; ATM = ataxia telangiectasia-mutated; DOCK = dedicator of cytokinesis; IL-2RG = IL-2 receptor gamma; JAK = Janus kinase; MHC = major histocompatibility complex; NEMO = nuclear factor-kappa-B essential modulator; PTPRC = protein tyrosine phosphatase, receptor type C; RAG = recombination activating gene; RFX = regulatory factor X; RFXANK= RFX containing ankyrin repeats; RFXAP = RFX-associated protein; RMRP = ribonuclease mitochondrial RNA-processing; SCID = severe combined immunodeficiency; STAT = signal transducer and activator of transcription; TYK = tyrosine kinase; and WASP = Wiskott-Aldrich syndrome protein. | | | |

Omenn syndrome may be understood as an autosomal recessive form of severe severe combined immunodeficiency (SCID) causing erythroderma, desquamation, alopecia, chronic diarrhea, failure to thrive, lymphadenopathy, eosinophilia, hepatosplenomegaly, and elevated serum IgE levels.

In a preferred embodiment, the patient suffers from X-linked agammaglobulinemia (XLA). In another preferred embodiment, the patient suffers from common variable immunodeficiency (CVID). In another preferred embodiment, the patient suffers from X-linked agammaglobulinemia (XLA) and from common variable immunodeficiency (CVID).

Optionally, the patient may also suffer from one or more viruses such as, e.g. human immunodeficiency virus (HIV), influenzae A, influenzae B, adenoviruses, rhinoviruses, coronaviruses etc.

In another preferred embodiment, the patient suffering from an immune deficiency is immunosuppressed, i.e., has a secondary immune deficiency (e.g. administered with an immunosuppressant drug).

The present invention also refers to an epitope-specific IgA against *Haemophilus influenzae* and/or *Streptococci pneumoniae* and/or *Neisseria meningitides* species for inhalation in a patient with (severe) immunodeficiency.

The invention is further explained by the following examples and figures and claims which are intended to illustrate the present invention.

### Brief Description of the Figure

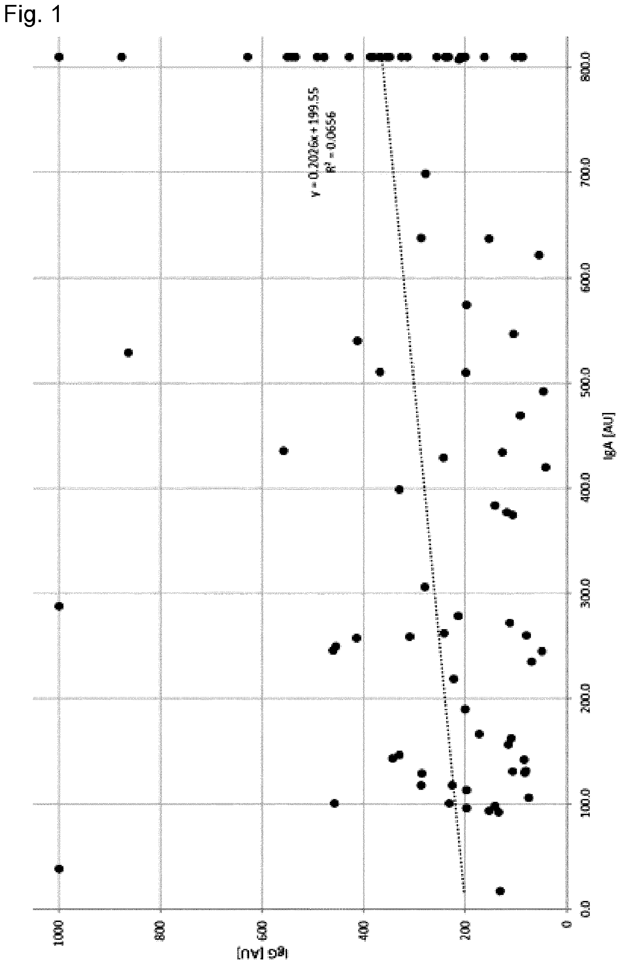

**Figure 1****:** Correlation between IgG and IgA specific antibodies against Pneumococcal antigens in healthy plasma donors (final concentration [U] SP IgG). This shows that in healthy humans, there are individuals with high specific IgG, those with high specific IgA and those having high specific IgG as well as IgA. Mixture could lead to a sufficiently high titer. Thus, there is often no need for donor selection for generating a hyperimunogenic solution. Accordingly, the method of the present invention is sufficient to provide a hyperimmunogenic product.

### Examples

### Example I: Obtaining (hyper)immune plasma from blood and plasma donors

Plasma or blood donors may be optionally immunized either with a vaccine containing polyribosyl ribitol phosphate (PRP) against *H. Influenzae* or a vaccine against S. *pneumonia* capsular carbohydrate antigens.

The PRP antigen of *H. influenzae* may be present in polyvalent vaccines (e.g. Pentvac® (Sanofi-Aventis), Infanrix or Infanrix-IPV or Infanrix hexa (GlaxoSmithKline GmbH & Co. KG) and may be used for boosting donors with an existing basic immunization. Therefore, a single shot of the vaccine may be placed according to the manufacturer's description intramuscularly. The immune respond may show an increased production of specific anti-PRP antibodies within the following 14 days. The immune response may be mostly present for at least 4 months.
The pneumococcal vaccines (e.g. Pneumovax® 23 (Sanofi-Aventis), Synflorix (GlaxoSmithKline GmbH & Co. KG), Prevenar 13® (Pfizer Pharma) are used for boosting donors with an existing basic immunization. Therefore, a single shot of the vaccine may be placed according to the manufacturer's description intramusculary.

The immune respond may show an increased production of specific anti-PRP antibodies within the following 10 days. The immune response may be mostly present for at least 8 months. In this 4 - 8 of a high titer immune response, it may be suitable to let those donors donate plasma according to the respective national guideline. In Germany, e.g., up to twice a week for a volume of 650 - 850 mL depending on the body weight.

### Example II: Detection of specific antibodies against H. influenzae or S. pneumonia

For the detection of antibodies against *H. influence,* microtiter plates were coated with 1 µg/mL methylated BSA (bovine serum albumin) in a 0.1 mol/L carbonate buffer pH 9.6 (overnight 4 °C, 100 µL/well). After three washing steps, a second coating with polyribosyl ribitol phosphate (PRP) at a concentration of 1 µg/mL was carried out (HIB-ELISA).

For the detection of antibodies against *S. pneumoniae,* microtiter plates were coated with 1 µg/mL Pneumovax® (MSD Sharp & Dohme GmbH, 85540 Haar, Germany) in a 0.1 mol/l carbonate buffer pH 9.6 (overnight 4 °C, 100 µL/well). After three washing steps, a second coating with PRP at a concentration of 1 µg/mL was carried out (Pneum-ELISA).

The coated plates were washed using PBS, 0.3 mol/L NaCl, 0.1 %(v/v) Tween 20. Before the sample incubation, the plates were washed three times with 200 µL/well. Samples were diluted for the HIB-ELISA 1:50, for the Pneum-ELISA in the range of from 1:200 to 1:4000. Samples were diluted using the washing buffer with added 3 % by weight Gelafusal (Serumwerk Bernburg AG, 06406 Bernburg Germany) and incubated for 90 min at room temperature with 100 µL/well for a double detection.

After three washing steps, the incubation with the HRP-conjugated anti-human-lgA at a concentration of 0.5 µg/mL followed for 90 min at room temperature. After three washing steps, the substrate solution (TMB, SeramunBlau® fast2, Seramun, Wolzig, Germany) followed for 20 min. The reaction was stopped by adding 100 µL of 1 mol/L H₂SO₄. Plates were measured at 450 nm. The results were calculated by using internal standards with a high titer. (Porstmann et al. Enzyme immunoassay techniques. An overview. Journal of Immunological Methods, 1992, 150:5-21).

Donors showed a high activity of both immunoglobulin classes IgG and IgA against either HIB or Pneumococci. This shows that a high proportion of donors may be vaccinated against both HIB and Pneumovax. The levels of available hyperimmune donors may be influenced by the environment, where they may be exposed to potential endemic infections.

### Example III: Preparation of an affinity matrix for the purification of specific antibodies against H. influenzae and/or S. pneumoniae

EAH Sepharose 4B was formed by covalent linkage of 1,6-diaminohexane to Sepharose 4B using an epoxy coupling method. EAH Sepharose 4B bears free amino groups at the end of 11-atom spacer arms, which are used to couple ligands containing carboxyl groups with the carbodiimide coupling method. The long hydrophilic spacer arm makes EAH Sepharose 4B particularly suitable for immobilization of small carbohydrate molecules. EHA Sepharose was prepared according to the manufacturer's instructions:
1.) EAH Sepharose 4B was supplied pre-swollen in 20 %(v/v) ethanol. The ethanol solution was decanted and washed with the required amount of matrix on a sintered glass filter (porosity G3) with distilled water adjusted to pH 4.5 with HCI, followed by 0.5 M NaCl (80 mL) in aliquots/mL sedimented matrix.
2.) The method for coupling is the carbodiimide method. Carbodiimides may be regarded as anhydrides of ureas. The N,N'-disubstituted carbodiimide promotes condensation between a free amino and a free carboxyl group to form a peptide link by acid catalyzed removal of water. The water soluble carbodiimide N-ethyl-N'-(3-dimethylaminopropyl) was used. It was found that, alternatively, also other agents could b used in this context such as, e.g., carbodiimide hydrochloride (EDC) or N-cyclohexyl-N'-2-(4'-methyl-morpho-linium) ethyl carbodiimide-p-toluene sulphonate (CMC). The reactions could be performed in accordance with manufacturer's instructions.
3.) The concentration of carbodiimide used experimentally was higher than the stoichometric concentration. The amount was usually 10-100 times higher than the concentration of spacer groups. A concentration of carbodiimide of 0.1 M was used. The coupling reaction with either the carbohydrates from the HIB-vaccine (Broncho-Vaxom® Vifor Pharma, Vifor Pharma Deutschland GmbH, 81379 Munich, Germany) or from Pneumovax at a concentration of 0.5 mg/mL each was performed in distilled water adjusted to pH 4.5 to 6.0 to promote the acid-catalyzed condensation reaction. The pH value of the reaction mixture decreased during the first hour of the coupling. The pH must thus be adjusted during this time by addition of dilute sodium hydroxide solution. Temperature and time dependence Coupling was completed within 12 hours at 4 °C. Acetic acid (1 M) was used as a suitable blocking agent. To remove the excess of uncoupled ligand that remains after coupling, the adsorbent was washed with high and low pH buffer solutions three times (Acetate buffer at 0.1 M, pH 4 and Tris buffer 0.1 M, pH 8.3 each containing 0.5 M NaCl were used.

### Example IV: Purification of specific antibodies IgA against H. influenzae and/or aS. pneumoniae

Immunoglobulin A (IgA) was isolated direct from plasma of from plasma fractions which were purified from IgG. When those fractions were used, the separation of the specific immunoglobulin classes in a first step was not needed. When the positive affinity chromatography was performed before the separation of the immunoglobulin classes, the separation of them was performed in a second step.

An example for the separation of (hyperimmune) IgA included subjecting a plasma pool to Cohn fractionation. This was followed by an IgG polishing step providing IgG and an IgA/IgM fraction. The last fraction is normally not used for the purification of other immunoglobulins for therapeutic approaches. The IgA/IgM fraction was subjected to affinity chromatography as described above and provides (hyperimmune) IgA.

An alternative example for the separation of (hyperimmune) IgA included subjecting an un-fractioned plasma pool subjected to affinity chromatography as described above. This provides an immunoglobulin fraction (including IgG and IgA) and a residual blood fraction which is further subjected to Cohn fractionation. The immunoglobulin fraction (including IgG and IgA) was subjected to a second affinity chromatography separating IgG and IGA and provides (hyperimmune) IgA.

The columns with the immobilized antigens/epitopes were used for the affinity chromatography. The column was washed with a buffer (PBS). The column was contacted with the body fluid (blood serum or a fraction thereof). The antibodies and antibody fragments specifically bind to the respective epitope bind to their targets in the affinity column. The column was washed again by a flow-through of buffer. Thereby unbound components including unbound antibodies and antibody fragments having no or merely a low affinity to the polypeptide are removed. Subsequently, the specific antibodies and antibody fragments were eluted either by acetic buffers (e.g. 0.1 mol/L glycine/HCI pH 2.0) or by 3 mol/L KSCN, or by 8 mol/L urea. Thereby, the specifically binding antibodies were isolated in high concentrations.

### Example V: IgA purification

The feed was diluted in equal parts with 1-fold PBS buffer. The feed could either consists of plasma, cryo-poor plasma or any plasma derived solution which is produced during plasma fractionation. The resin (*CaptureSelect IgA*) was equilibrated with 1-fold PBS buffer and was used in a chromatography column. A use in batch-mode was also possible. The solution was loaded onto the chromatography column. The elution was performed with a 0.1 mol/L glycine pH 2.2. The eluate was immediately neutralized in the fraction sample by 0.1 mol/L Tris. 1.0 mol/L citric acid is then used to CIP the column, which was then equilibrated afterwards using 1-fold PBS again.

### Example VI: Animal model

The primary objective of such a study was to evaluate the dose of specific human IgA antibodies against *H. influenzae* and/or *S. pneumoniae* in an inhalation therapy. In a three-armed feasibility animal study specific IgA against the *H*. *influenzae or S. pneumoniae* and no therapy are compared. The read out in this study was the colonization rate.

IgA was prepared as described above from plasma of normal healthy plasma donors. The donors have previously been screened for the presence of specific antibodies against *H. influenzae* and/or *S. pneumoniae* using the ELISA described above. IgA was enriched from those donations. The mice (8 per group) were pretreated with a solution a either *H. influenzae* or *S. pneumoniae* solutions (10⁶ CFU/mL) by a nasal wash. IgA-enriched plasma-fraction was stored frozen until use in single does, each at 250 µL with 1 mg/mL. The IgAs was administered by a nose drops into the animal upper respiratory tract 24 h after the pretreatment. Eight male animals are used per group:
A Disease control group remains completely untreated after bacteria application. Two IgA-treatment groups infected with either *H. influenzae* or *S. pneumoniae.* The colonization rate was observed by RT-PCR (Falla et al., PCR for capsular typing of Haemophilus influenzae. J Clin Microbiol 1994, 32:2382-2386.). The treated groups showed a significant reduced colonization rate until 1 week after treatment.

## Claims

1. A method for isolating a fraction of immunoglobulin A (IgA) specifically binding to one or more carbohydrates specific for the surface of one or more bacteria species from a body fluid, wherein the method comprises the following steps:
(i) providing:
the body fluid containing one or more immunoglobulins specifically binding to the one or more carbohydrates; and
a solid support containing one or more immobilized carbohydrates;
(ii) contacting the body fluid of step with the solid support and allowing binding of the one or more of immunoglobulins to the one or more carbohydrates immobilized on the solid support,
(iii) removing the remaining body fluid containing components not bound to the one or more carbohydrates immobilized on the solid support in step (ii) from the solid support and optionally washing the solid support; and
(iv) eluting the one or more of immunoglobulins specifically binding to the one or more carbohydrates immobilized on the solid support from the solid support,
wherein the method further comprises at least one step of separating IgA from other immunoglobulins, in particular from immunoglobulin G (IgG).

2. The method of claim 1, wherein the body fluid is blood plasma or a fraction thereof.

3. The method of any of claims 1 or 2, wherein the body fluid containing one or more of immunoglobulins specifically binding to the one or more carbohydrates is obtained from a subject previously exposed to the one or more carbohydrates, optionally to the one or more bacteria species bearing the one or more carbohydrates on their surface.

4. The method of any of claims 1 to 3, wherein the at least one step of separating IgA from other immunoglobulins is conducted before the body fluid is contacted with the solid support.

5. The method of claim 4, wherein the body fluid is the IgA/IgM fraction of Cohn fractionation and optionally further removing immunoglobulin M (IgM).

6. The method of any of claims 1 to 3, wherein the at least one step of separating IgA from other immunoglobulins is conducted in the eluate obtained in step (v), in particular the eluate subjected to Cohn fractionation and optionally further removing immunoglobulin M (IgM).

7. The method of any of claims 1 to 6, wherein the one or more carbohydrates are surface carbohydrates of *Haemophilus influenzae, Streptococcus pneumoniae, Neisseria meningitides,* or a combination of two or more thereof.

8. The method of any of claims 1 to 7, wherein the one or more carbohydrates are polyribosyl ribitol phosphate (PRP), in particular PRP containing 5 to 50 units of PRP moieties.

9. The method of any of claims 1 to 8, wherein the one or more bacteria species are bacteria associated with one or more respiratory diseases, in particular are selected from the group consisting of *Haemophilus influenzae, Streptococcus pneumoniae, Neisseria meningitides,* and a combination of two or more thereof.

10. A fraction of IgA obtainable from a method of any of claims 1 to 9.

11. A pharmaceutical composition comprising the fraction of IgA of claim 10 and at least one pharmaceutically acceptable carrier.

12. The pharmaceutical composition of claim 11, wherein the pharmaceutical composition is a spray, an aerosol, a nose drop, an unguent, an ointment, a gel, or a microcapsule.

13. The fraction of IgA of claim 10 or the pharmaceutical composition of any of claims 11 or 12, wherein the IgA specifically binds to one or more carbohydrates specific for the surface of one or more bacteria species selected from the group consisting of *Haemophilus influenzae, Streptococcus pneumoniae, Neisseria meningitides,* and a combination of two or more thereof,
for use in a method for treating or preventing a respiratory disease associated with an infection with *Haemophilus influenzae, Streptococcus pneumoniae, Neisseria meningitidis,* or a combination of two or more thereof.

14. The fraction of IgA or the pharmaceutical composition for use of claim 13, wherein the fraction of IgA or the pharmaceutical composition is administered to the patient's respiratory tract and/or as an aerosol

15. The fraction of IgA or the pharmaceutical composition for use of any of claims 13 or 14, wherein the patient suffers from an immune deficiency, in particular an immune deficiency selected from the group consisting of X-linked agammaglobulinemia (XLA), common variable immunodeficiency (CVID), hyper-IgM syndrome with AID or UNG deficiencies, hyper-IgM syndrome with CD40 deficiency, hyper-IgM syndrome with CD40 ligand deficiency, selective antibody deficiency with normal immunoglobulins, selective IgA deficiency, transient hypogammaglobulinemia of infancy, ataxia-telangiectasia cartilage-hair hypoplasia, combined immunodeficiency with inadequate but not absent T-cell function and normal or elevated immunoglobulins, hyper-lgE syndrome, MHC antigen deficiencies, severe combined immunodeficiency, Wiskott-Aldrich syndrome, and combination of two or more thereof.
